# EUROPEAN PATENT SPECIFICATION

(11) **EP 0 417 988 B1**
(45) Date of publication and mention of the grant of the patent: **18.11.1993**
(21) Application number: 90309801.0
(22) Date of filing: 07.09.1990
(51) Int. Cl.: A61M 5/28, A61M 5/178

(54) **Bipartite injector device**
Zweiteilige Injektionsvorrichtung
Dispositif d'injection en deux parties

(30) Priority: 12.09.1989 US 405989; 20.11.1989 US 439333
(43) Date of publication of application: 20.03.1991
(73) Proprietor: F.H. FAULDING & CO. LTD., Parkside South Australia 5063 (AU)
(72) Inventor: Rohrbough, John, Scottsdale, Arizona 85259 (US)
(74) Representative: Moon, Donald Keith

(56) References cited:
- US-A- 3 523 531
- US-A- 4 576 211
- US-A- 4 581 016
- US-A- 4 607 671

## Description

The present invention relates to a bipartite injector device and to a syringe mixer and bipartite injector device in combination.

Many syringe mixer and/or injector constructions are known in the prior art, some having simple rib and groove or like interconnections of parts, e.g. as shown in United States Patents 3,563,373 to Paulson and 3,570,486 to Engelsher et al. Similar showings appear in United States Patent 3,523,531 to Burke and 4,411,163 to White. However, the prior art devices are generally deficient in that they do not permit of low cost production and assembly of accurately aligned parts and fluid tight interconnection thereof. Prior art devices may also carry a risk of accidental skin puncture by a user.

United States Patent 2,490,552 to Smith for example shows a syringe having an axially shiftable single hollow needle with opposed pointed ends arranged to permit the user to push the exposed pointed end inwardly until the internal pointed end punctures a seal to flow connect the needle with a prefilled medicament chamber, to prepare the syringe for use, but disadvantageously in slow and cumbersome manner, and at risk of accidental skin puncture or other injury to the user when pushing the exposed needle and inwardly against the seal, as well as at risk of contamination of the medicaments, the user and the environment.

United States Patent 3,397,694 to Ogle shows a medicament powder charged syringe having a single hollow needle, with its inner end separated from the powder by a stopper, and its outer pointed end in the neck of a medicament liquid charged, ram containing, vial having a plug facing the pointed needle end, enabling the liquid to be forced by the ram against the plug until punctured by the needle, and then pass through the needle to unseat the stopper and mix with the powder, followed by vial removal for use of the syringe.Instead of a needle, the syringe and vial may have luer lock connections, with the vial using a bypass plug to enable the liquid to reach the powder when forced by the ram, the syringe then being used with a dispensing needle having a corresponding luer lock connection.

Disadvantageously, the constructions of this Ogle patent are complicated, expensive, require many different and precisely interfitting parts, and must be used with a special type liquid charged vial, and thus must withstand without leakage the internal hydraulic pressures generated in operating the vial ram to effect transfer of the liquid to the syringe.

United States Patent 4,516,967 to Kopfer shows a medicament liquid charged syringe whose needle is pushed to puncture the seal of a medicament powder charged vial, whereupon the liquid is forced from the syringe into the vial to mix with the powder and then the admixture is withdrawn back into the syringe, disadvantageously requiring slow and cumbersome two-way liquid transfer to achieve mixing and syringe preparation.

United States Patent 4,619,651 to Kopfer et al shows a syringe transfer system similar to the above Ogle and Kopfer patents but in this case the syringe, whose needle is attached by a luer lock connection, is used with a special type vial whose neck has a double seal forming an intermediate holding chamber.

United States Patent 3,542,023 to Ogle; United States Patent 3,547,122 to Rinser et al; United States Patent 3,563,373 to Paulson; and United States Patent 3,570,486 to Engelsher et al, variously show analogous syringes having a single long hollow needle with opposed pointed ends arranged for charging the syringe upon puncturing the seal of an associated vial via a given pointed end of the needle.

United States Patent 4,648,532 to Green shows a powder charged dental preparation capsule having a front nozzle and a rear ram, and containing a liquid filled pillow inwardly of the nozzle, such that on pushing a rod into the nozzle to puncture the pillow, the released liquid admixes with the powder, enabling the ram to force the mixture out through the nozzle.

It would be a significant advance over the prior art to provide injector device whose parts could be simply assembled utilising a snap fit arrangement. This would provide for low cost production and assembly of parts which could be accurately aligned and provide fluid tight connection, yet retain the ability to separate the various pieces. It is also important with at least some types of syringes that the component parts are not able to inadvertently become detached from each other. This is particularly so where the medicament being handled is hazardous to those administering the substance. It has been found, with some prior art devices, that transportation and handling of the syringes prior to use can cause component parts which should be in relatively sealed contact with each other to move relative to each other breaking the seal. In other instances that seal can inadvertently be broken should the syringe plunger be withdrawn relative to the outer shell of the syringe, even to a relatively small extent, resulting in leakage of the medicament or contamination thereof. For this reason it has been the practice, at least in some instances, to permanently fix component parts together. This adds significantly to the cost of the syringe, which is undesirable.

It would be a further significant advance over the prior art if a syringe mixer and injector device could be provided of detachable parts for transfer of a medicament liquid from a charging vial to a medicament solid or liquid in a receiving vial for admixture therein without retransfer to the charging vial, yet easily and rapidly, under safe and sterile conditions, and permitting the part associated with the filled receiving vial to be detached and connected directly and without modification to a dispensing device.

It is an object of the present invention to overcome, or at least alleviate, one or more of the difficulties or deficiencies of the prior art.

Accordingly in a first aspect of the present invention there is provided a bipartite injector device comprising
an elongate hollow outer shell having a front closed end, a rear open end, a right circular cylindrical inner wall forming a chamber within said shell, a hub coaxial with said inner wall extending through said closed end from said chamber to a forward position exterior of said shell, a bore extending through said hub, a coaxial seat surrounding said bore within said chamber, a spout formed by the end of the hub exterior of the shell, and a first part of a snap fit locking assembly formed on the end of said inner wall adjacent said closed end, and
a right circular cylindrical inner sleeve having a front end, a coaxially mounted tubular spike, and a rear end, a second part of said snap fit locking assembly being formed on said inner sleeve front end for engagement with said first part to mount said inner sleeve in cantilever manner within said chamber, said front end including sealing means to sealingly engage with said seat when said sleeve is operatively mounted in said shell, said spike having a pointed end which faces rearwardly and terminates inwardly of the rear open end of the outer shell sufficiently to protect the spike from unintended human contact, a coaxial flow passage extending through said spike and said inner sleeve, the radially outer diameter of the sleeve being less than the diameter of the inner wall so that when said sleeve is operatively mounted in said shell an annular space is formed between said inner wall and said sleeve, said sleeve having a connection socket in said rear end, said spike protruding into said socket,
said injector device being adapted to receive a cylindrical medicament vial in said open end of said shell, said vial having a hollow cylindrical wall with a wall thickness which is less than the radial distance between said inner wall and said sleeve, said vial being slidable relative to said shell and, guided for movement by coaction with said inner wall and/or said sleeve, said vial having an open end closed by a penetrable stopper, said stopper having a projection thereon shaped and configured to connect with and be held in said socket, said stopper being axially slidable within said vial said spike being configured such that its pointed end penetrates said stopper when said vial is operatively connected to said injector device to provide for fluid tight flow between said vial and said spout through the spike, flow passage, and bore.

The two part snap fit locking assembly is preferably formed by a radially inwardly directed constriction ring formed near the front end of the inner wall, and a radially outwardly directed locking flange on the front end of the sleeve, the flange being arranged to snap lock with the constriction ring to cantilever mount the sleeve to the shell.

The bipartite injector device according to this aspect formed of an outer shell and inner sleeve connected in cantilever manner to the shell for fluid tight flow of a medicament between a vial connected to one end of the device and a spout at the other end of the device, may, in turn be connected to a separate charging, mixing or dispensing device. The bipartite injector device may thus be fabricated from conventional materials and components and assembled in simple and inexpensive manner. Moreover since the spike terminates inwardly from the outer end of the inner sleeve, the needle is protected from unintended human contact.

The shell may include a generally radial front wall forming its closed end, and the sleeve front end is preferably sized and arranged to abut that wall when the rim and groove are in locking engagement.

The spike may be in the form of an elongate needle having a front collar flange, the sleeve having a coaxial needle mounting portion which mounts the needle in fluid tight manner to the sleeve.

In a preferred embodiment of the present invention the needle mounting portion has a forwardly facing central circular recess at the sleeve front end, a rearwardly facing central circular extension and a passage from the recess to the extension, the recess, extension and rim being coaxial. The collar flange is arranged for coaxial mounting on the extension, and the recess is arranged to receive the seat coaxially. The needle mounting portion has connector means and the collar flange has counterpart connector means to connect the needle to the sleeve at the extension.

The sleeve may have an annular excavation radially between its flange and recess and extending from its front end toward its extension to define a concentric compression compensation space to aid local compression displacement of the rim through the ring for snap fit locking in the groove.

In an alternative embodiment of the present invention, the needle mounting portion has a central circular hollow recess formation with a circular shoulder, the formation, shoulder and rim being coaxial, and the formation and shoulder sized to receive and engage the collar flange with the formation peripherally embracing it and the shoulder axially locating it to mount it on the seat.

The recess formation may include a forwardly facing larger diameter central circular neck recess at the sleeve front end outwardly bounded by a forward thin wall neck extending rearwardly from the sleeve flange, and a rearwardly facing smaller diameter central circular shank recess outwardly bounded by a thick wall shank extending rearwardly from, and separated by a shoulder from, the neck. The shoulder is arranged to locate the collar flange rearwardly of the foward-most neck extent, and the neck is sized to aid the rim compression displacement through the ring to lock in the groove.

The shell has a nozzle outwardly confining the spout at its closed end and arranged, in use, to connect thereto to a separate charging, mixing or dispensing device for fluid tight flow between the vial and separate device via the needle and spout. The nozzle may have a luer lock connector mating with a counterpart connector on the separate device for their releasable interconnection. The socket may have threads mating with those on the vial stopper.

Preferably, the shell and sleeve are plastic, e.g. formed as injection molded pieces, for snap fit cantilever connection to provide the bipartite device, and the needle is metal.

It is also important to note that the snap lock cantilever mounting arrangement by means of which the sleeve is mounted in the shell ensures that the spike or needle is substantially rigidly and axially mounted within the shell. Thus, when the vial is operatively inserted into the open end of the shell the sharp end makes penetrating contact with the penetrable barrier in the stopper. This is important in emergency situations and to avoid wastage, and also because the spike end is inward of the rear end of the shell and thus the user is unable to guide the stopper into engagement with the spike end.

In a further aspect of the present invention there is provided a syringe mixer and bipartite injector device, comprising a bipartite injector of the type described above and an adapter, each having front closed and rear open end and a protected fluid pathway extending longitudinally therethrough from the inner end to the outer end thereof;
the bipartite injector comprising an external nozzle in its front closed end thereof;
the adapter comprising a counterpart connection nozzle in the front closed end thereof, defining the inner terminus of its pathway, the external nozzle and counterpart connection nozzle being arranged to interconnect releasably the injector and adapter to flow connect their pathways in protected fluid tight condition, the adapter having a guideway extending from the rear open end towards the front closed end thereof, adapted, in use, to receive and guide for movement relative thereto a cylindrical medicament vial having an upper end closed by a penetrable stopper; and
a connection socket in the rear open end thereof adapted to connect stationarily the penetrable stopper thereat, and a tubular spike protruding into the socket and terminating inwardly from the rear open end sufficiently to protect the spike from unintended human contact and arranged, in use, to penetrate the stopper to flow connect the vial with the pathway in fluid tight communication.

The syringe mixer and injector device may function as follows. The recessed tubular spike in each socket penetrates the stopper of a vial when connected to that socket, to charge the injector connected vial with the contents of the adapter connected vial. Then the injector nozzle is disconnected from the adapter nozzle and connected directly and without modification to a dispensing device having a like connectable nozzle to that of the adapter.

The device may be used with a receiving vial charged with a medicament solid or liquid, and a charging vial charged with a medicament liquid for one-way transfer to the receiving vial for admixing the medicaments therein without retransfer to the charging vial, and without risk of accidental skin puncture or other injury to the user from any exposed needles, or of contamination of the medicaments, the user or the environment.

The device may function to provide for administration quickly and safely a preset dosage of two separate medicaments readily combined at the time of use, under improved conditions of sterility, and providable in a compact, storable form, and which may be fabricated from conventional materials and components in simple and inexpensive manner.

Desirably, the nozzle and counterpart nozzle have mating luer lock connection formations to interconnect releasably the injector and adapter.

In particular, each socket may define an axial cylindrical recess and each spike may define an axial hollow cylindrical tubular portion centrally arranged coaxially with its respective recess. Each spike desirably is cantilevered and has a pointed free end and a base end which is integral with the adjacent portion of the corresponding end of the injector and adapter through which the respective pathway extends. Also, each socket preferably has an internal thread connection formation adapted, in use, to mate with a counterpart external thread connection formation on the stopper of a corresponding vial to be connected thereto.

The shell and sleeve of the bipartite injector device and adapter are formed as plastic injection molded pieces.

Significantly, in this aspect of the present invention, no portion of the spikes need be made of metal, which is important in the case of plastic short pointed spikes in that while they will be sufficiently pointed to achieve easy and rapid penetration of standard rubber or like material stoppers on the vials, they will not be so sharp as to present a danger of puncturing the skin of the user.

Moreover, in fabricating the plastic spikes, there is no need to undergo the trouble and expense of providing separate metal needles for incorporation into the injector and adapter and for interconnecting the other portions of the injector and adapter thereto, e.g. by staking technique, nor of cutting or grinding the metal needle outer ends to form them into sharp pointed ends to penetrate the vial stoppers, and then cleansing the needles to remove undesired metal particulates generated during the cutting or grinding, before such staking.

Also, since the short plastic spikes are formable integrally with the adjacent portions of the injector and adapter, they will be much sturdier and more precisely centered in their sockets, e.g. to tolerances controlled by the injection molding process, for more accurate coaxial alignment with the stopper bores of the vials to achieve easy, rapid and safe attachment of the stoppers to the sockets without misalignment of the spikes and stopper bores.

In contrast thereto, a syringe unit having a metal needle is subject to the risk of the needle not being assembled in the construction in perfectly centered alignment for engaging the stopper bore of the associated medicament vial, whereupon the needle may not find the center of the stopper when screwed into the socket of the syringe unit containing the misaligned needle. Once off center, as the vial stopper is rotated into the socket, the needle will bury itself into the thick sidewall of the stopper and not flow connect with the vial contents, thereby rendering the syringe unit inoperable, a result to be avoided in attempting an emergency injection. Use of extra force to achieve flow connection raises the further risk of shattering the vial and injuring the user's hand.

When the injector and adapter of the device according to the present invention are interconnected, these short spikes will form single pointed free end cantilevered tubular extensions in their sockets facing in opposite directions, firmly supported, e.g. molded, at their base ends to the remainder of the injector and adapter and permanently housed entirely, within the confines of the surrounding barrel formed by the corresponding outer end of the injector and adapter defining the given socket.

The vials may be of standard known type, with one vial being charged with a preset dosage of a liquid, e.g. sterile water, as medicament L, and the other vial being charged with a preset dosage of either another liquid or a solid, such as a particulate powder or tablet, etc., e.g. an emergency drug required to be administered during cardiac arrest, quickly, safely and under sterile conditions, as medicament P compatable with, e.g. dissolvable in, medicament L of the vial.

Hence, the device according to this aspect of the present invention may be used as a manually operated liquid-liquid (wet-wet) or liquid-solid (wet-dry powder) two compartment mixing and injecting system achieving all the above noted objects. It is especially useful for combining the medicaments by one-way transfer from the charging vial to the plunger vial without retransfer to the charging vial, instead attaining mixing only in the plunger vial, e.g. by mere manual agitation.

The injector and adapter, and the associated vials may be compactly prepackaged as disposable, one-time use, sterile items, e.g as an emergency kit, with the injector and adapter connected as a preassembled unit having removable protective caps (not shown) on their outer ends, with similar caps being provided on the open ends of the two vials.

This permits the steps of opening the package, removing the caps, pushing the vials onto the injector and adapter for transfer and mixing, detaching the filled plunger vial and injector from the adapter and charging vial, and attaching the standard dispensing device, all to be effected easily and rapidly, without danger to the user from contact with toxic or hazardous medicaments or substances or exposed needle points, or the need to modify the injector to accommodate the standard dispensing device, and at minimum risk of contaminating the sterile medicaments or the environment.

The one-way, one-step transfer and mixing system contemplated is thus safer to use than conventional systems, since transfer and mixing are achieved in a completely closed two compartment arrangement, in which from start to finish the user is never exposed to an unprotected needle, let alone required to bend the needle to snap it off from the injector to permit attachment of a separate dispensing device.

The hazard of cuts from working with a device having an exposed needle has become even more dangerous in terms of increased exposure in present day hospital and home care environments to contaminating substances such as those related to HIV (Human Immunodeficiency Virus) and AIDS (Acquired Immune Deficiency Syndrome).

The present invention will now be more fully described with reference to the accompanying drawings. It should be understood, however, that the description following is illustrative only and should not be taken as a restriction on the generality of the invention described above.
Figure 1 is a schematic sectional view of a bipartite injector device according to one, e.g. large size, embodiment of the invention, with a medicament vial connected thereto;
Figures 2 and 3 are exaggerated partial views of the Figure 1 device, showing the snap fitting of the sleeve to the shell;
Figure 4 is a schematic sectional view of a bipartite injector device according to another, e.g. small size, embodiment of the invention, with such a vial connected thereto; and
Figures 5 and 6 are similar to Figures 2 and 3, but show the snap fitting of the sleeve to the shell of the Figure 4 device;
Figure 7 is a schematic exploded view of a syringe mixer and injector device according to an embodiment of the present invention, showing the injector and adapter, each associated with a medicament containing vial;
Figure 8 is a schematic view of the device of Figure 7, showing the injector and adapter connected to each other and to their associated medicament containing vials for charging the contents of the adapter associated vial via the adapter and injector to the injector associated vial.

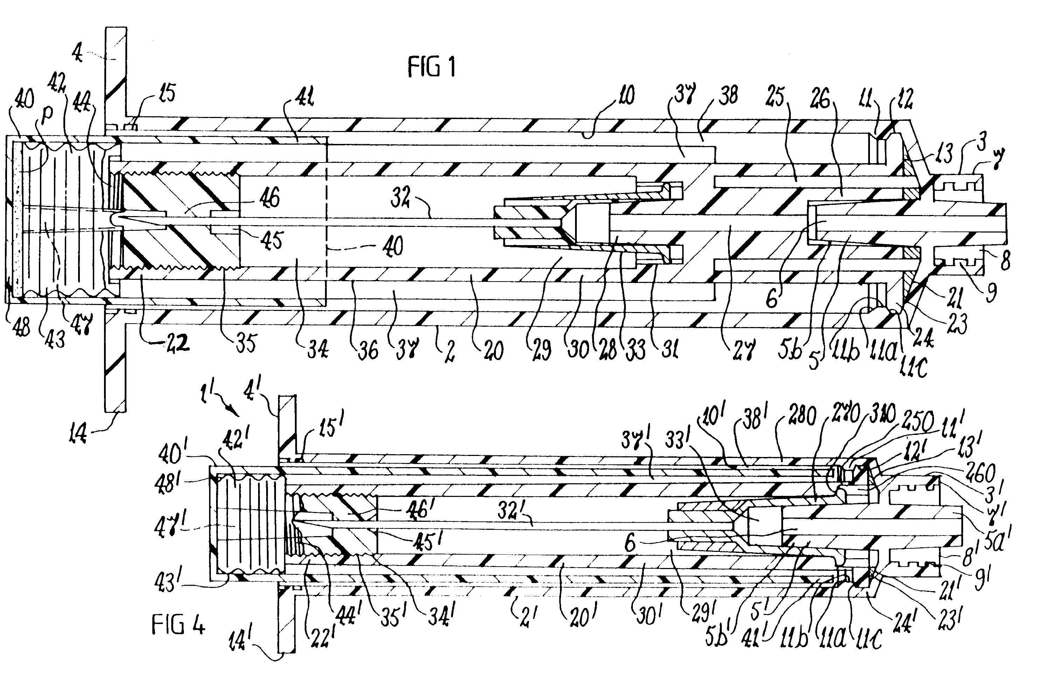

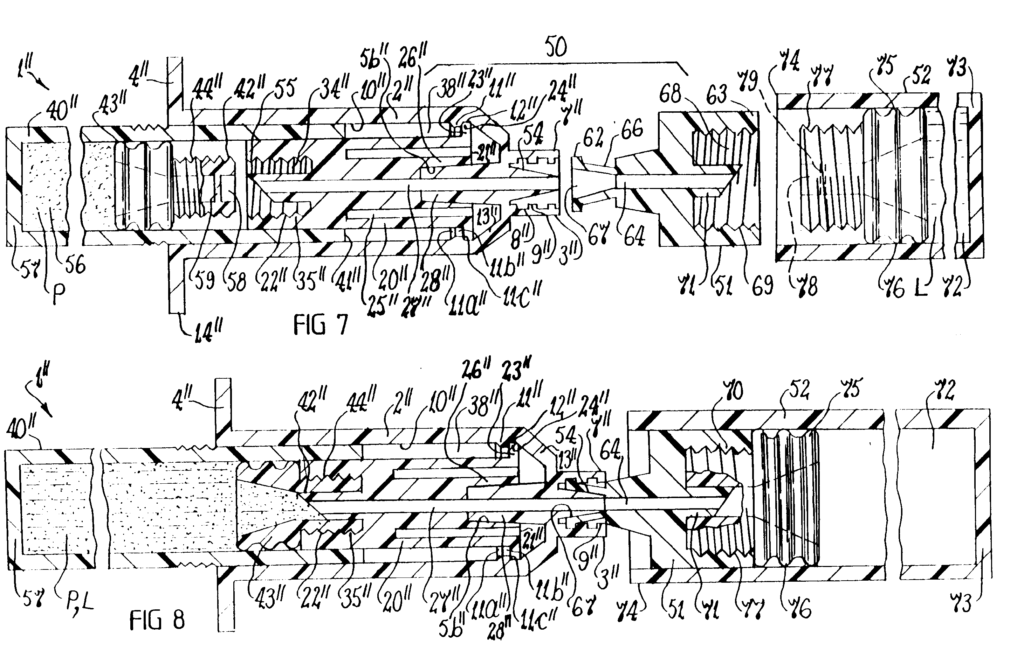

Referring to the drawings, and initially to Figure 1, a bipartite injector device 1 is shown, including a longitudinal, e.g. cylindrical, hollow outer shell 2 and a cylindrical inner sleeve 20, plus a conventional cylindrical medicament powder or liquid containing receiving vial in the form of a plunger vial 40 at the rear end of device 1. Such may be used with an adapter connectable to the front end of device 1 and having its own medicament liquid containing charging vial (not shown) as described more fully in relation to Figure 7 below.

Shell 2 has a front closed end 3, a circular rear open end 4 and a circular central hub 5. Hub 5 extends through closed end 3 from its external circular, e.g. tapered, spout 5a to its internal circular, e.g. tapered, seat 5b in the shell interior, and has a central bore 6 extending internally from spout 5a to seat 5b. An external nozzle 7 at closed end 3 outwardly confines spout 5a and has a recess 8 provided with a connector, e.g. a conventional luer lock such as male luer lock 9, to connect nozzle 7 to a separate device having a counterpart connector, such as a female luer lock, e.g. a charging device formed of an adapter connected to a charging vial or a dispensing device such as an injection needle (not shown).

Cylindrical inner wall 10 of shell 2 forms an interior to receive coaxially via end 4 and guide vial 40 for relative longitudinal and rotational movement, whose open end 41 is closed by shiftable cylindrical, needle penetrable, stopper 42 having ring 43 to seal its, eg. powder, medicament P content.

Inner wall 10 has a radially inwardly directed circular constriction ring 11 adjacent closed end 3 and a radially inwardly facing circular locking groove 12 defined axially thereon between ring 11 and the radial front wall 13 forming closed end 3. Ring 11 is formed of a hollow, e.g. about 30 degree angle, frustoconical insert ramp 11a, a hollow cylindrical center span 11b, and an opposed hollow, e.g. about 45 degree angle, frustoconical lock ramp 11c.

Wall 10, ring 11, groove 12 and seat 5b are circular in cross section, concentric and coaxial, and bore 6 extends along the center axis of shell 2.

Rear end 4 has lateral flanges 14 formed as finger grips to aid handling of device 1 in use, and a groove formation 15 to receive an aseptic friction fit closure cap (not shown) to keep device 1 sterile before use with vial 40. A similar cap (not shown) protects spout 5a and nozzle 7 at front end 3.

Sleeve 20 has a front cantilever mounting end 21 and a rear free end 22. Front end 21 has a radially outwardly directed circular flange 23 terminating peripherally in a circular compression rim 24, sized and arranged to coact with ring 11 and groove 12 to connect front end 21 of sleeve 20 in cantilever manner to front end 3 of shell 2. Front end 21 also has an annular excavation 25 and a forwardly facing central circular, e.g. tapered, recess 26 communicating via central internal passage 27 with a rearwardly facing central circular, e.g. tapered, extension 28.

Excavation 25 is located radially between flange 23 and recess 26, extending from front end 21 toward extension 28 to define a concentric compression space to aid local compression displacement of rim 24 through ring 11 for snap fit locking in groove 12. Recess 26 is sized and arranged to receive seat 5b of hub 5 in fluid tight condition to communicate bore 6 with passage 27. The remainder of the sleeve 20 interior at extension 28 forms an annular space 29 within rearward cylindrical extension 30 having internal threads 31.

Hollow needle 32, e.g. a conventional standard, regular bevel (B-D I.V. 166A x 1 1/2'') metal needle, is disposed in cylindrical extension 30 with its externally threaded front circular, e.g. tapered, collar flange 33 mounted in fluid tight condition on central extension 28 via internal threads 31 on extension 30 at annular space 29, to communicate with passage 27. This mounting also positions the rear pointed end of needle 32 coaxially in socket 34, in rear end 22 at the remote portion of extension 30, and centrally of socket internal threads 35, within sleeve circular outer wall 36.

Rim 24, space 25, recess 26, extension 28, space 29, extension 30, socket 34 and outer wall 36 are circular in cross section, concentric and coaxial, and passage 27 extends along the center axis of sleeve 20.

Recess 26, central extension 28, space 29 and cylindrical extension 30 provide a central needle mounting portion between rim 24 and socket 34, so that when rim 24 firmly locks in groove 12, recess 26 automatically firmly seats against seat 5b, and threads 31 selectively adjustably engaging threaded collar flange 33 automatically locate needle 32 to seat flange 33 automatically firmly against extension 28, for fluid tight flow between needle 32 and spout 5a via passage 27 and bore 6.

Matching tapers for seat 5b, recess 26, extension 28 and collar flange 33, and preset positioning of needle 32 per threaded collar flange 33 and threads 31, assure fluid tight connection of the pertinent needle 32 and sleeve 20 portions, as rim 24, ring 11 and groove 12 are sized, located and matched for tight snap fit connection of sleeve 20 to shell 2.

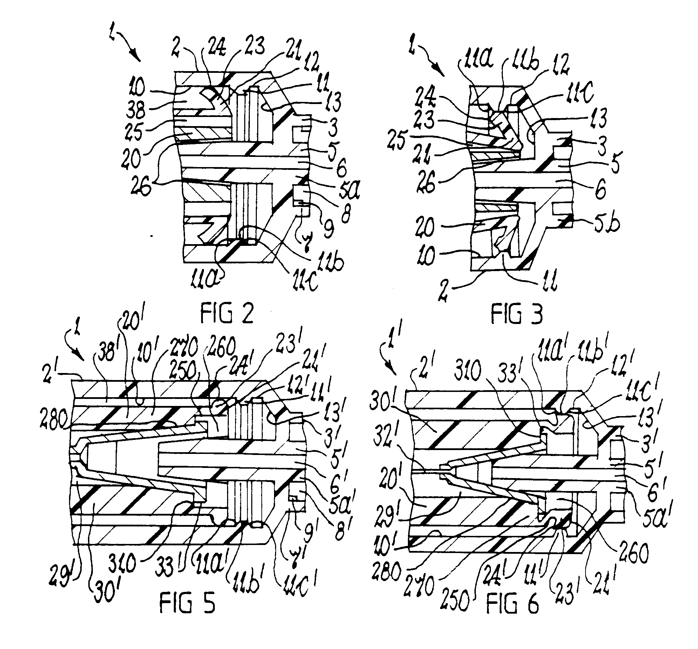

As shown in Figures 2 to 3, rim 24 is precisely sized for compression displacement through ring 11 and snap fit locking in groove 12, so that sleeve 20 may be readily connected to shell 2 by unskilled labor. As these two pieces are moved increasingly telescopingly together, e.g. under manual force using a simple jig, rim 24 is compressed against, e.g 30 degree, shallow incline insert ramp 11a, promoting its movement to and across cylindrical span 11b, aided by space 25 to permit compensating radially inward temporary deformation of rim 24 and flange 23, due to the inherent resiliency of the material, e.g. plastic, of which sleeve 20 is made.

When rim 24 reaches, e.g. 45 degree, steep incline lock ramp 11c, its snap fit movement into groove 12 is assisted, since its loaded compression force is rapidly released along ramp 11c as it expands to its original radial dimension and tightly and firmly stationarily engages groove 12, as shown in Figure 1. By conforming front end 21 of sleeve 20 at flange 23 to the counterpart internal shape of front end 3 of shell 2 at radial wall 13, by appropriate sizing and shaping of these parts, they may be placed in firm stationary abutment with flange 23 acting against wall 13 under the snap fit cantilever locking connection force between rim 24 and groove 12.

Thus, ring 11 and groove 12 form a circular snap fit cantilever connector formation in shell 2 adjacent closed end 3, and flange 23 and rim 24 form a counterpart snap fit cantilever connector formation on sleeve front end 21, the two formations being sized and arranged for snap fit interlocking to mount sleeve 20 coaxially in shell 2 with front end 21 in cantilever stationary connection with closed end 3.

Shell 2 and sleeve 20 are preferably formed as injection molded plastic pieces, to promote their snap fit cantilever locking and fluid tight connection, and enable use of low cost dies of simple design, yet of precise dimension and shape, to fabricate these two pieces with conforming parts accurately mating to achieve their desired concentric and coaxial alignment relationship that is essential to proper alignment with vial 40 during use. For example, shell 2 and sleeve 20 may be sized for 10 mL to 50 mL dosages with concordantly sized vials.

As shown in Figure 1, internal threads 35 in socket 34 connect stopper 42 of vial 40 stationarily thereat when open end 41 is screwed therein via stopper external threads 44. This causes the pointed end of needle 32 to align coaxially with stopper entrance 45, penetrate seal 46, enter stopper bore 47 and flow communicate with vial chamber 48. By grasping flanges 14, vial 40 may be pushed between shell inner wall 10 and sleeve outer wall 36, preferably having circumferentially spaced longitudinal guide ribs 37, along guideway 38.

Ribs 37 selectively radially size the annular gap of guideway 38, i.e. between the outer radius of sleeve ribs 37 and inner radius of shell wall 10, to conform to the vial 40 radial dimensions, while conserving material and lightening the structure without detracting from the robust structural integrity of sleeve 20, in that the circumferential arc portions between ribs 37 constitute excavated spaces on outer wall 36. Ribs 37 inhibit wobble between shell 2, sleeve 20 and vial 40, and misalignment between needle 32 and stopper entrance 45.

A larger size device 1 may be used for a smaller size dosage in appropriate cases, as by using a 10 mL size device 1 with a half filled 10 mL size vial 40, i.e. having a 5 mL of medicament P, e.g. with stopper 42 shifted half way into open end 41, to eliminate the need for a 5 mL size device 1 (and closure caps) and a 5 mL size vial 40 and stopper 42. End 41 of the half filled vial 40 may be closed by a cap, similar to the use of caps to cover open end 4 and nozzle 7 and spout 5a.

When the half filled vial is inserted in guideway 38 and stopper 42 screwed into socket 34 to cause needle 32 to enter stopper entrance 45 and puncture seal 46, vial 40 may be pushed further into device 1 while stopper 42 is stationarily connected to socket 34, to force medicament P from the filled rear half of vial 40 via needle 32, passage 27 and bore 6 to spout 5a, e.g. when connected to a separate dispensing device.

Figures 4 to 6 show an alternative embodiment in which analogous parts to those of Figures 1 to 3 have prime (') designations. While device 1 of Figures 1 to 3 is used in large dosage sizes, device 1' of Figures 4 to 6 is used in smaller sizes, e.g. for 0.5 mL to 3 mL dosages, with needle 32' being an analogous standard, regular bevel (B-D I.V. 196A x 1 1/2') metal needle, and shell 2' and sleeve 20' plastic, e.g. injection molded, pieces.

Device 1' has a shell 2' of parts 3' to 15' and a sleeve 20' of parts 21' to 24', 29' to 30' and 32' to 38', generally the same as those of device 1, but of smaller size, for use with an analogous vial 40' of parts 41' to 48' generally the same as those of vial 40, but of concordantly smaller size. However, the needle mounting portion of sleeve 20' is modified relative to the analogous parts of device 1, as device 1' is of such small size that it would be too difficult and costly in practice to provide it with the construction of device 1.

Device 1' has a needle mounting portion with an enlarged central circular hollow recess formation and circular shoulder that are coaxial with rim 24' and sized to receive and engage collar flange 33' with the recess formation peripherally embracing flange 33' and the shoulder axially locating it to mount it directly on seat 5b'. The recess formation includes a forwardly facing larger diameter central circular neck recess 260 at sleeve front end 21' outwardly bounded by a forward thin wall cylindrical neck 250 extending rearwardly from sleeve flange 23', and a rearwardly facing smaller diameter central circular shank recess 280 outwardly bounded by a thick wall cylindrical shank 270 extending rearwardly from neck 250 and separated therefrom by shoulder 310.

As shown in Figure 4, neck recess 260, shank 270, shank recess 280 and shoulder 310 are sized and arranged to locate collar flange 33' axially rearwardly of the forwardmost extent of neck 250 in fluid tight fit directly on and against seat 5b' to communicate needle 32' with shell bore 6', analogous to recess 26, passage 27, extension 28 and threads 31 of device 1.

As shown in Figures 5 to 6, neck 250 is sized to aid local compression displacement of rim 24' through ring 11' for snap fit locking in groove 12', analogously to space 25 of device 1.

By precise sizing of neck 250, shoulder 310 and shank 270, and thus of recess 260 and recess 280, relative to collar flange 33', and of rim 24' relative to groove 12' and seat 5b', when rim 24' locks in groove 12' collar flange 33' automatically seats firmly against seat 5b' and is held thereat by shank 270 and shoulder 310 connected via neck 250 with rim 24', acting in one axial direction thereagainst, and by seat 5b' acting in the opposite axial direction thereagainst.

In both devices 1 and 1', when the vial is screwed into the socket, the pushing and rotational movement of the vial inhibits possible rotation of the sleeve relative to the shell, as this movement applies friction force urging the rim against the groove and the flange against the radial wall.

In device 1, this force also urges recess 26 against seat 5b and needle 32 against extension 28 for like inhibition of possible rotation of sleeve 20 and needle 32. In device 1', this force also urges shank 270 and shoulder 310 against collar flange 33' and the latter against seat 5b', at the same time preventing axial compression of thin wall neck 250. Neck 250 is sufficiently thick to withstand axial tension between rim 24' and shoulder 310 in keeping collar flange 33' on seat 5b'.

Thus, the construction of devices 1 and 1' permits their low cost fabrication and their low cost manual assembly by unskilled labor into a bipartite unit composed of two individual and separate pieces, interconnected by snap fit, and not welded or otherwise integrally interconnected, and incorporating therewith a standard commercially available type needle.

The parts of the two separate pieces are precisely formed, sized, shaped and arranged, relative to each other to maintain their snap fit cantilever interconnection, with their parts in accurate centered alignment, and especially with the given, i.e. cantilever mounted, standard needle centered relative to the sleeve socket, which centering is critical for vial stopper center puncturing without misalignment or other mishap, especially in an emergency when medicament charging, transfer and/or dispensing, e.g. via separate standard charging and/or dispensing devices, must be effected rapidly and safely both as regards the patient and the user of the device.

The parts are desirably sized to position the needle pointed end coaxially in the socket slightly inwardly of the sleeve open end sufficiently to protect the needle from unintended human contact thereat. Due to the precise fluid tight fit of the associated internal parts of device 1 and device 1', the flow path from the vial through the needle via passage 27 and bore 6 in device 1, or via bore 6 alone in device 1', is completely protected and entirely free of dead spaces. Devices 1 and 1' may be provided as one-time use discardable items.

Of course, the circular connector formation formed by the flange and rim on sleeve front end 21 or 21' may be a continuous circular formation of uninterrupted circular profile, or a discontinuous circular formation, eg. notched at its periphery like a gear to form a tooth-like interrupted circular profile, yet will still achieve the stated snap fit interlocking.

Since in devices 1 and 1' the ring and groove are formed on the shell, and the flange and rim are formed on the sleeve, as integral parts, they will be more precisely centered and axially positioned, e.g. to tolerances controlled by the injection molding process, for more accurate coaxial alignment of the needle mounted thereon with the stopper entrance of the vial, to achieve easy, rapid and safe screwing of the stopper into the socket without needle and stopper bore misalignment.

On the other hand, once off centre, as the vial stopper is rotated into the socket, the needle will bury itself into the thick sidewall of the stopper and not flow connect with the vial contents, thereby rendering the unit inoperable, a result to be avoided in attempting an emergency injection. Use of extra force to achieve flow connection in such case raises the added risk of shattering the vial and injuring the user's hand.

Referring to the further alternative embodiment illustrated in Figure 7, in which analogous parts to those of Figure 1 to 3 have double prime ('') designations, a syringe mixer and injector device 50 is shown, including a bipartite injector device 1'' including a longitudinal, e.g. cylindrical hollow outer shell 2'' and a cylindrical inner sleeve 20'' and an adapter 51, plus an associated conventional medicament powder or liquid containing receiving vial in the form of a plunger vial 40'' for injector 1'' and an associated medicament liquid containing charging vial 52 for adapter 51.

Shell 2'' has a front closed end 3'' and a rear open end 4'', and defines a protected, e.g. central, fluid pathway extending longitudinally therethrough from front end 3'' to rear end 4''.

Shell 2'' has a connection nozzle 7'' provided with a nozzle recess 8'' containing a tapered, e.g. central, spout 54 defining the inner terminus of the protected fluid pathway and a connection formation, such as a luer lock tab 9'' arranged to form a male connection formation for interconnecting releasably with mating parts on adapter 51 to flow connect their pathways in protected fluid tight condition, as noted below.

Cylindrical inner wall 10'' of shell 2'' forms an interior to receive coaxially via end 4'' and guide vial 40'' for relative longitudinal and rotational movement, whose open end 41'' is closed by shiftable cylindrical, spike penetrable, stopper 42'' having ring 43'' to seal its, e.g. powder, medicament P content.

Inner wall 10'' has a radially inwardly directed circular constriction ring 11'' adjacent closed end 3'' and a radially inwardly facing circular locking groove 12'' defined axially thereon between ring 11'' and the radial front wall 13'' forming closed end 3''. Ring 11'' is formed of a hollow, e.g. about 30 degree angle, frustoconical insert ramp 11a'', a hollow cylindrical center span 11b'', and an opposed hollow, e.g. about 45 degree angle, frustoconical lock ramp 11c''.

Wall 10'', ring 11'', groove 12'' and seat 5b'' are circular in cross section, concentric and coaxial, and bore 6'' extends along the center axis of shell 2''.

Sleeve 20'' has a front cantilever mounting end 21'' and a rear free end 22''. Front end 21'' has a radially outwardly directed circular flange 23'' terminating peripherally in a circular compression rim 24'', sized and arranged to coact with ring 11'' and groove 12'' to connect front end 21'' of sleeve 20'' in cantilever manner to front end 3'' of shell 2''. Front end 21'' also has an annular excavation 25'' and a forwardly facing central circular, e.g. tapered, recess 26'' communicating via central internal passage 27'' with a rearwardly facing central circular, e.g. tapered, extension 28''.

Sleeve 20'' has a guideway 38'' extending therefrom toward front closed end 3'', e.g. formed as an internal recess of annular or hollow cylindrical shape, to receive and guide,e.g. coaxially, plunger vial 40'' for movement both longitudinally and rotationally relative thereto. Sleeve 20'' also has a connecting socket 34'' defined therein and provided with internal threads 35'', plus a relatively short, e.g. central, tubular spike 55 defining the outer terminus of the protected fluid pathway and protruding, e.g. coaxially, into socket 34'' and terminating inwardly from rear open end 4'' sufficiently to protect spike 55 from unintended human contact at socket 34''. Lateral flanges 14'' may be formed on rear open end 4'' as user finger grips.

The injector associated plunger vial 40'' may be of conventional type, e.g. a cylindrical member having a chamber 56 charged with a preset dosage of a medicament liquid or solid P, eg. a powder, a closed rear end 57 and an open front end 41'' sealed by a shiftable internal cylindrical stopper 42''. Stopper 42'' is screwed therein via external threads 44'', plus an internal, e.g. central, bore 58 containing a penetrable seal 59 adjacent the forwardmost portion of its tip.

The exterior of plunger vial 40'' may contain score line graduations to define dosage volume or other indicia.

Plunger vial 40'' and guideway 38'' are sized and, e.g. coaxially, arranged for mating coaction to permit open end 41'' of cylindrical plunger vial 40'' to be inserted slidably, e.g. coaxially, into the counterpart recess of guideway 38'' at injector rear open end 4'' for longitudinal reciprocation as well as rotation of plunger vial 40'' on injector 2''. Also, stopper 42'' and socket 34 are sized and, e.g. coaxially arranged for mating coaction to permit external threads 44'' on the top of stopper 42'' to be screwed onto internal threads 35'' in socket 34'' sufficiently for spike 27'' to penetrate seal 59, e.g. coaxially, and flow connect pathway and chamber 56 in protected fluid tight connection, as plunger vial 40'' is rotated relative to injector 2'' (Figure 8).

Since plunger vial 40'' initially contains a relatively small volume charge of medicament P, stopper 42'' will normally be initially located in recessed position remote from open end 41'', such that plunger vial 42'' may be inserted in guideway 38'' without spike 55 penetrating seal 59 (Figure 7).

Likewise, adapter 51 has an inner end 62 and an outer end 63, and a protected, e.g. central, fluid pathway 64 extending longitudinally therethrough from inner end 62 to outer end 63.

Adapter inner end 62 has a counterpart connection nozzle 66, provided with a tapered, e.g. central, recess or entry 67 defining the inner terminus of pathway 64 and a connection formation, such as a luer lock tab 67, arranged to form a female connection formation for interconnecting releasably with the male connection formation formed by spout 54'', and luer lock tab 9'' in recess 8'' of shell 2'', e.g. coaxially therewith, to flow connect pathways 27'' and 64, in protected fluid tight condition and entirely free of dead spaces (Figure 8).

Adapter outer end 63 has its own guideway 68 extending therefrom toward inner end 62, e.g. formed as a cylindrical, e.g. coaxial, outer surface thereof, to receive and guide, e.g. coaxially, charging vial 52 for movement both longitudinally and rotationally relative thereto. Outer end 63 also has its own connecting socket 69 defined therein and provided with internal threads 70, plus a relatively short, e.g. central, tubular spike 71 defining the outer terminus of pathway 64 and protruding, e.g. coaxially, into socket 69 and terminating inwardly from outer end 63 sufficiently to protect spike 71 from unintended human contact at socket 69.

The adapter associated charging vial 52 may likewise be of conventional type, e.g. a cylindrical member having a chamber 72 charged with a preset dosage of a medicament liquid L, a closed rear end 73 and an open front end 74, sealed by a shiftable internal cylindrical stopper 75 having external seal rings 76 on its main diameter body portion engaging the interior surface of chamber 72, and external threads 77, e.g. coaxially, on its reduced diameter cylindrical tip portion spaced inwardly from the interior surface of chamber 72, plus an internal, e.g. central, bore 78 containing a penetrable seal 79 adjacent the forwardmost portion of its tip (shown in phantom in Figure 7).

Charging vial 62 and guideway 68 are likewise sized and, e.g. coaxially, arranged for mating coaction to permit open end 74 of cylindrical charging vial 52 to be inserted slidably, e.g. coaxially, onto the counterpart surface forming guideway 68 at adapter outer end 63 for longitudinal reciprocation as well as rotation of charged vial 52 on adapter 51. Also, stopper 75 and socket 69 are sized and, e.g. coaxially, arranged for mating coaction to permit external threads 77 on the top of stopper 75 to be screwed onto internal threads 70 in socket 69 sufficiently for spike 71 to penetrate, e.g. coaxially, seal 78 and flow connect pathway 64 and chamber 73 in protected fluid tight condition, as charging vial 52 is rotated relative to adapter 51 (Figure 8).

Likewise, stopper 75 will be stationarily connected by its threads 77 to threads 70 of socket 69, yet by reason of its shiftable disposition in chamber 72, reciprocation of charging vial 52 will cause stopper 75 to move longitudinally therealong relative to open end 74 since charging vial 52 initially contains a relatively large volume charge of medicament liquid L, stopper 75 will normally be initially located adajcent the open end, such that more or less immediately upon inserting charging vial 52 onto guideway 68, spike 69 can penetrate seal 79 of stopper 75 (Figure 7).

Desirably, in injector 1'' and adaptor 51, respectively, each socket 34'', 69 defines an axial cylindrical recess and each short spike 55, 71 defines an axial hollow cylindrical tubular portion centrally arranged coaxially with its respective recess. Also, each spike 55, 71, is cantilevered and has a pointed free end and a base end which is integral with the adjacent portion of the corresponding end of injector 50 and adapter 51.

Thus, when counterpart nozzle 66 of adapter 51 is connected to nozzle 7'', and plunger vial 40'' is mounted on guideway 38'' of injector 2'', and charging vial 52 is mounted on guideway 68 of adapter 51, charging vial 69 may be rotated to screw stopper 75 into socket 69 to cause spike 71 to penetrate seal 78 and then plunger vial 40'' may be rotated to screw stopper 42'' into socket 34'' to cause spike 55 to penetrate seal 59, whereby to achieve tandem flow concentration of plug bore 78, pathway 64, and plug bore 58 in protected fluid tight condition, and form a continuous, yet separable, interior fluid conduit system between the vials, entirely free of dead spaces.

Then, charging vial 52 may be pushed towards adapter 51 to transfer liquid L from chamber 72 through plug bore 78, pathways 64 and 27'', and plug bore 58 into chamber 57, thereby causing plunger vial 20'' to be retracted outwardly from injector 1'' as liquid L fills chamber 57 and admixes with solid or liquid P already present therein (Figure 8). These attached components may be manually shaken, if needed, to assure complete admixing of medicaments P,L in plunger vial 40'' but the basic procedure of combining medicaments P,L is achieved by a one-way, one-step transfer of liquid L from vial 52 to vial 40'' through adapter 51 and injector 1'', without any retransfer of the combined medicaments P,L back to vial 52.

Thereafter, adapter 51 (and vial 52) may be detached from injector 4'' (and vial 40'') and a separate standard dispensing device, having a tapered entry and luer lock tab, i.e. forming a female connection formation counterpart to the male connection formation on injector nozzle 7'', may be immediately and directly attached to nozzle 7'', without intervening modification, enabling injector 1'', carrying plunger vial 40'' (now filled with the desired preset dosage volume of admixed medicaments P,L) on outer end 4'', and the attached dispensing device on inner end 3'', to be used forthwith to dispense the admixed medicaments.

The separate standard dispensing device may be for instance a three-way stopcock or protected needle for injection into a latex resealable I.V. injection port of an intravenous injection unit, or the like, or any other dispensing device, e.g. for non-injectable use of the medicament mixture, having a mating connection formation to that of injector nozzle 6, such as a mating luer lock connection formation as described above.

These luer lock formations permit easy and rapid connection and release onto injector nozzle 6 of counterpart nozzle 34 of adapter 51 and the corresponding connection formation of the separate dispensing device, and may be of standard size. These luer lock connection formations are easily and rapidly connected and disconnected, e.g. by twisting clockwise to connect them and counterclockwise to disconnect them.

Parenthetically, a certain commercially available medicament mixing and transfer syringe unit utilizes, in conjunction with other components, an injector element having a permanently attached sharp pointed exposed needle, such that once slow and cumbersome transfer and retransfer steps have been undertaken to achieve mixing, disadvantageously the user must take extra time and care to bend back and forth the exposed needle until it snaps off, thereby subjecting the user to the danger of cutting a finger on the sharp bevel portion of the needle, before a separate dispensing device can be attached thereto for dispensing the admixed medicaments.

On the other hand, spikes 55, 71 need only be sufficiently long to penetrate vial seals 59, 79 to provide communication between vial chamber 72 and vial chamber 57. Thus, they are advantageously recessed a pronounced distance from the entrance to sockets 34'', 69 and in turn from outer ends 4'', 63 to protect these relatively short spikes 55, 71 from unintended human contact, yet permit easy and, rapid penetration of seals 59, 79 by mere twisting of vials 40'' ,52 relative to injector 2'' and adapter 63.

All portions of injector 1'' and adapter 51, respectively include nozzles 7'' and their luer lock connection formations, and their spikes 55, 71 may be made of suitable rigid plastic such as polycarbonate, e.g. by injection molding technique, especially with each spike being integrally interconnected to the adjacent interior socket portion of the injector and adapter defining an interior protected pathway therein, e.g. molded-in-place as cantilevered short tubular structures thereon and in recessed relation to the socket opening, so as to form a one-piece member injector 1'' and adapter 51.

However, while the structural portions defining nozzles 7'', 66 may be formed of injection molded parts integral with the remainder of the respective injector shell 1'' and adapter 51, optionally the structure portions defining these respective nozzles 6'', 34 may be provided as separate such parts, e.g. of a common standard size, and then mounted on and connected, e.g. by sonic welding technique, to the remainder of the respective injector 1 and adapter 51, which may be of any appropriate size, among a number of different sizes, depending on the medicament volume dosage to be admixed and administered, yet fashioned to accommodate the common standard size nozzle 7'', 34 thereon.

The specification and drawings are set forth by way of illustration and not limitation, and various modifications and changes may be made therein without departing from the scope of the invention which is to be limited solely by the scope of the claims.

## Claims

1. A bipartite injector device (1) comprising
an elongate hollow outer shell (2) having a front closed end (3), a rear open end (4), a right circular cylindrical inner wall forming a chamber within said shell, a hub coaxial with said inner wall extending through said closed end from said chamber to a forward position exterior of said shell, a bore (6) extending through said hub, a coaxial seat (5b) surrounding said bore within said chamber, a spout (5a) formed by the end of the hub exterior of the shell, and a first part (11, 12) of a snap fit locking assembly formed on the end of said inner wall adjacent said closed end, and
a right circular cylindrical inner sleeve (20) having a front end (21), a coaxially mounted tubular spike (55), and a rear end (22), a second part (24) of said snap fit locking assembly being formed on said inner sleeve front end for engagement with said first part to mount said inner sleeve in cantilever manner within said chamber, said front end including sealing means to sealingly engage with said seat when said sleeve is operatively mounted in said shell, said spike having a pointed end which faces rearwardly and terminates inwardly of the rear open end of the outer shell sufficiently to protect the spike from unintended human contact, a coaxial flow passage (27) extending through said spike and said inner sleeve, the radially outer diameter of the sleeve being less than the diameter of the inner wall so that when said sleeve is operatively mounted in said shell an annular space is formed between said inner wall and said sleeve, said sleeve having a connection socket in said rear end, said spike protruding into said socket,
said injector device being adapted to receive a cylindrical medicament vial (40) in said open end of said shell, said vial having a hollow cylindrical wall (41) with a wall. thickness which is less than the radial distance between said inner wall and said sleeve, said vial being slidable relative to said shell and guided for movement by coaction with said inner wall and/or said sleeve, said vial having an open end closed by a penetrable stopper (42), said stopper having a projection thereon shaped and conf igured to connect with and be held in said socket, said stopper being axially slidable within said vial said spike being conf igured such that its pointed end penetrates said stopper when said vial is operatively connected to said injector device to provide for fluid tight flow between said vial and said spout through the spike, flow passage, and bore.

2. A bipartite injector device according to claim 1 wherein the first part of said snap fit locking assembly is formed by a radially inwardly directed constriction ring (11) formed near the front end of the inner wall, and the second part of said snap fit locking assembly is formed by a radially outwardly directed locking flange (23) on the front end of the sleeve, the flange being arranged to snap lock with the constriction ring to cantilever mount the sleeve to the shell when the sleeve is operatively inserted into the shell.

3. A bipartite injector device according to claim 1 or 2 wherein the spike is in the form of an elongate needle (32) having a front collar flange (33), and the sleeve has a coaxial needle mounting portion which mounts the needle in fluid tight manner to the sleeve.

4. A device according to claim 2 wherein the shell has a generally radial front wall forming the front closed end thereof, and the sleeve flange is sized and arranged to abut stationarily against the front wall when the flange is in snap fit locking engagement with the ring.

5. A device according to claim 3 wherein the needle mounting portion includes a forwardly facing central circular recess at the sleeve front end, a rearwardly facing central circular extension and said flow passage extends therethrough from the recess to the extension, the collar flange being arranged for coaxial mounting on the extension, and the recess being arranged to receive the seat coaxially.

6. A device according to claim 5 wherein the needle mounting portion has connector means adjacent the extension and the collar flange has counterpart connector means to connect the needle to the sleeve at the extension.

7. A device according to claim 3, wherein the sleeve has an annular excavation (25) radially between the sleeve flange and recess and extending from the sleeve front end toward the extension to define a concentric compression compensation space to aid local compression displacement of the flange through the ring for snap fit locking in the groove.

8. A device according to claim 3 wherein the needle mounting portion includes a central circular hollow recess formation (26) having a circular shoulder, the recess formation, shoulder and flange being coaxial, and the recess formation and shoulder being sized to receive and engage the collar flange with the recess formation peripherally embracing the collar flange and the shoulder axially locating the collar flange to mount the collar flange directly on the seat.

9. A device according to claim 8 wherein the recess formation includes a forwardly facing larger diameter central circular neck recess (260) at the sleeve front end outwardly bounded by a forward thin wall neck portion (250) extending rearwardly from the sleeve flange, and a rearwardly facing smaller diameter central circular shank recess (280) outwardly bounded by a thick wall shank portion (270) extending rearwardly from the neck portion and separated therefrom by the shoulder, the shoulder being arranged to locate axially the collar flange rearwardly of the forwardmost extent of the neck portion, and the neck portion being sized to aid local compression displacement of the flange through the ring for snap fit locking in the groove.

10. A device according to any one of the preceding claims, wherein the shell has an external nozzle (7) at its closed end outwardly confining the spout and arranged, in use, to connect a separate charging, mixing or dispensing device thereto for fluid tight flow between a vial and that separate device through the needle and spout.

11. A device according to claim 10 wherein the nozzle has a luer lock connector (9) to mate with a counterpart luer lock connector on that separate device for releasable interconnection thereof.

12. A device according to any one of the preceding claims, wherein the socket (34) has internal thread means (35) to mate with counterpart external thread means (44) on the stopper of the corresponding vial to be connected thereto.

13. A device according to any preceding claim wherein the shell and sleeve are formed as plastic injection molded pieces for snap fit cantilever connection.

14. A bipartite injector device (1) comprising
a longitudinal hollow outer shell (2) including a front closed end (2), a rear open end (4), a cylindrical inner wall forming an interior adapted, in use, to receive coaxially through the open end and guide for movement relative there to a cylindrical medicament vial (40) closed by a penetrable stopper (42), a central hub (5) extending through the closed end from an external hub spout (5a) to an internal hub circular seat (5b) in the shell interior and having a bore (6) extending therethrough from the spout to the seat, and a circular snap fit cantilever connector formation (11, 12) in the shell interior adjacent the closed end, the inner wall, connector formation and seat being coaxial, and
a cylindrical inner sleeve (20) including a front cantilever mounting end (21) having a counterpart circular snap fit cantilever connector formation (24), a central needle mounting portion (28) and a rear free end (22) having a cylindrical connection socket (34) adapted to connect stationarily such stopper thereat, and a hollow needle (32) having a front circular collar flange (33) and a rear pointed end, the sleeve, counterpart connector formation, needle mounting portion and socket being coaxial,
the sleeve being sized for location in the shell sufficiently radially inwardly of the inner wall to permit insertion of the vial therebetween, the connector formation and counterpart connector formation being sized and arranged for snap fit interlocking to mount the sleeve coaxially in the shell with the sleeve front end in cantilever stationary connection with the shell closed end, and the needle mounting portion being arranged to mount the needle coaxially and in fluid tight condition relative to the bore and sleeve to flow communicate the collar flange with the bore and to dispose the needle pointed end coaxially in the socket to penetrate such stopper for protected fluid tight flow between the vial and spout through the needle and bore.

15. A syringe mixer and bipartite injector device, comprising a bipartite injector according to any preceding claim and an adapter (51), each having front closed and rear open end and a protected fluid pathway extending longitudinally therethrough from the inner end to the outer end thereof;
the bipartite injector comprising an external nozzle in its front closed end thereof;
the adapter comprising a counterpart connection nozzle (66) in the front closed end thereof, defining the inner terminus of its pathway, the external nozzle and counterpart connection nozzle being arranged to interconnect releasably the injector and adapter to flow connect their pathways in protected fluid tight condition, the adapter having a guideway extending from the rear open end towards the front closed end thereof, adapted in use, to receive and guide for movement relative thereto a cylindrical medicament vial having an upper end closed by a penetrable stopper; and
a connection socket (69) in the rear open end thereof adapted to connect stationarily the penetrable stopper thereat, and a tubular spike (71) protruding into the socket and terminating inwardly from the rear open end sufficiently to protect the spike from unintended human contact and arranged, in use, to penetrate the stopper to flow connect the vial with the pathway in fluid tight communication.

16. A device according to claim 15 wherein the nozzle and counterpart nozzle have mating luer lock connection formations to interconnect releasably the injector and adapter.

17. A device according to claim 15 or 16 wherein each socket defines an axial cylindrical recess and each spike defines an axial hollow cylindrical tubular portion centrally arranged coaxially with its respective recess.

18. A device according to claim 17 wherein each spike is cantilevered and has a pointed free end and a base end which is integral with the adjacent portion of the corresponding end of the injector and adapter through which the respective pathway extends.

19. A device according to claim 18 wherein each socket has an internal thread connection formation adapted, in use, to mate with a counterpart external thread connection formation on the stopper of a corresponding vial to be connected thereto.

20. A device according to claim 19 wherein the shell and sleeve of the bipartite injector device and adapter are formed as plastic injection molded pieces.

## Patentansprüche

1. Zweiteilige Injektionseinrichtung (1) mit einem länglichen, hohlen äußeren Gehäuse (2), das ein vorderes geschlossenes Ende (3) ; eine gerade, kreisförmig zylindrische Innenwand, die in dem Gehäuse eine Kammer begrenzt; eine zu der Innenwand koaxiale Nabe, die sich aus der Kammer durch das geschlossene Ende hindurch zu einer vorderen, außerhalb des Gehäuses liegenden Stelle erstreckt; eine durch die Nabe verlaufende Bohrung (6); einen die Bohrung innerhalb der Kammer umgebenden koaxialen Sitz (5b); einen von dem außerhalb des Gehäuses liegenden Ende der Nabe gebildeten Konus (5a) sowie einen ersten Teil (11, 12) einer Rastverbindungsanordnung aufweist, die an einem Ende der Innenwand neben dem geschlossenen Ende ausgebildet ist, und mit
einer geraden, kreisförmig zylindrischen inneren Hülse (20), die ein vorderes Ende (21); einen koaxial befestigten, rohrförmigen Dorn (55); ein hinteres Ende (22) sowie einen zweiten Teil (24) der Rastverbindungseinrichtung aufweist, die an dem vorderen Ende der inneren Hülse zum Verrasten mit dem ersten Teil vorgesehen ist, um die innere Hülse in der Kammer in einer einenends befestigten, frei auskragenden Weise zu halten, wobei das vordere Ende mit Dichtungsmitteln versehen ist, um mit dem Sitz dichtend in Eingriff zu kommen, wenn die Hülse in dem Gehäuse gebrauchsfähig befestigt ist, und der Dorn ein zugespitztes Ende aufweist, das nach rückwärts weist und gegenüber dem hinteren offenen Ende des äußeren Gehäuses nach innen zurückversetzt endet, weit genug, um den Dorn gegen unbeabsichtigte menschliche Berührung zu schützen, und einen koaxialen Durchflußkanal (27) enthält, der durch den Dorn und die innere Hülse hindurchführt, wobei der radial außen liegende Durchmesser der Hülse kleiner ist als der Durchmesser der Innenwand, damit zwischen der Innenwand und der Hülse ein Ringspalt gebildet ist, wenn die Hülse gebrauchsfähig in das Gehäuse eingesetzt ist, und die Hülse in dem hinteren Ende einen Anschlußsockel trägt und die Nadel in den Sockel hineinragt,
wobei die Injektionseinrichtung so gestaltet ist, daß sie in dem offenen Ende des Gehäuses eine zylindrische Medikamentenampulle (40) aufnimmt, die eine hohle zylindrische Wand (41) mit einer Wandstärke aufweist, die kleiner ist als der radiale Abstand zwischen der Innenwand und der Hülse und die gegenüber dem Gehäuse verschieblich und durch Zusammenwirken mit der Innenwand und/oder der Hülse beweglich geführt ist, und die ein offenes, mittels eines durchstoßbaren Stopfens (42) geschlossenes Ende aufweist, wobei der Stopfen einen Fortsatz trägt, der so gestaltet und bemessen ist, und daß er mit dem Sockel verbindbar und darin zu halten ist und daß er in der Ampulle axial verschieblich ist, und wobei der Dorn so gestaltet ist, daß sein zugespitztes Ende durch den Stopfen hindurchdringt, wenn die Ampulle gebrauchsfähig mit der Injektionseinrichtung verbunden ist, um einen nach außen fluiddichten Kanal zwischen der Ampulle und dem Stutzen über die Nadel, den Strömungskanal und die Bohrung herzustellen.

2. Zweiteilige Injektionseinrichtung nach Anspruch 1, bei der der erste Teil der Rastverbindungseinrichtung von einem radial nach innen vorspringenden Einschnürungsring (11), der nahe dem vorderen Ende der Innenwand ausgebildet ist,und bei der der zweite Teil der Rastverbindungseinrichtung von einem radial nach außen gerichteten Rastflansch (23) an dem vorderen Ende der Hülse gebildet ist, wobei der Flansch dazu dient, mit dem Einschnürring zu verrasten, um die Hülse in dem Gehäuse einenends eingespannt frei auskragend zu halten, wenn die Hülse gebrauchsfertig in das Gehäuse eingesetzt ist.

3. Zweiteilige Injektionseinrichtung nach Anspruch 1 oder 2, bei der der Dorn die Gestalt einer länglichen Nadel (32) mit einem vorderen kragenförmigen Flansch (33) aufweist und die Hülse mit einem koaxialen Nadelbefestigungsteil versehen ist, über den die Nadel nach außen fluiddicht mit der Hülse verbunden ist.

4. Einrichtung nach Anspruch 2, bei der das Gehäuse eine im wesentlichen radial verlaufende Vorderwand aufweist, die das vordere geschlossene Ende bildet,und bei der der Flansch der Hülse so bemessen und angeordnet ist, daß er stationär gegen die Vorderwand anstößt, wenn der Flansch hinter dem Ring eingerastet ist.

5. Einrichtung nach Anspruch 3, bei der der Nadelbefestigungsteil eine nach vorne weisende, mittige, kreisförmige Vertiefung an dem vorderen Ende der Hülse und einen nach hinten zeigenden kreisförmigen Fortsatz aufweist, wobei sich der Strömungsweg von der Ausnehmung durch den Fortsatz hindurch erstreckt, der kragenförmige Flansch zur koaxialen Befestigung auf der Verlängerung dient und die Ausnehmung dazu vorgesehen ist, den Sitz koaxial aufzunehmen.

6. Einrichtung nach Anspruch 5, bei der der Nadelbefestigungsteil Verbindungsmittel aufweist, die bei dem Fortsatz angeordnet sind, und bei der der kragenförmige Flansch ein Verbindungsgegenstück umfaßt, um die Nadel mit der Hülse mittels des Fortsatzes zu verbinden.

7. Einrichtung nach Anspruch 3, bei der die Hülse mit einem ringförmigen Spalt (25) versehen ist, der radial zwischen dem Flansch der Hülse sowie der Ausnehmung liegt und der sich von dem vorderen Ende der Hülse in Richtung auf den Fortsatz erstreckt, um einen konzentrischen Kompressions-Kompensationsraum zu bilden, um eine örtliche Kompressionsbewegung des Flansches beim Durchgang durch den Ring zwecks Verrastung in der Nut zu unterstützen.

8. Einrichtung nach Anspruch 3, bei der der Nadelbefestigungsteil eine mittige, kreisförmige, hohle Ausnehmung (26) mit einer kreisförmigen Schulter umfaßt, wobei die Ausnehmung,die Schulter und der Flansch zueinander koaxial sind,und bei der die Ausnehmung sowie die Schulter so bemessen sind, daß sie den kragenförmigen Flansch aufnehmen und fassen, wobei die Ausnehmung den kragenförmigen Flansch am Umfang umgreift und die Schulter den kragenförmigen Flansch axial plaziert, damit der kragenförmige Flansch unmittelbar auf dem Sitz gehalten ist.

9. Einrichtung nach Anspruch 8, bei der die Ausnehmung eine an dem vorderen Ende der Hülse gelegene, nach vorne zeigende zentrale kreisförmige Halsausnehmung (260), die durch einen vorderen dünnen Wandhalsabschnitt (250) begrenzt ist, der sich, ausgehend von dem Hülsenflansch, nach hinten erstreckt, sowie eine nach hinten weisende zentrale kreisförmige Schaftausnehmung (280) mit kleinerem Durchmesser umfaßt, die nach außen durch einen dicken Schaftwandteil (270) begrenzt ist, der von dem Halsteil ausgehend sich nach hinten erstreckt und von diesem durch eine Schulter getrennt ist, wobei die Schulter dazu vorgesehen ist, den kragenförmigen Flansch hinter der vordersten Erstreckung des Halsteils axial zu lokalisieren und der Halsteil so bemessen ist, daß er eine lokale Kompressionsbewegung des Flansches beim Durchgang durch den Ring zum Einrasten in der Nut unterstützt.

10. Einrichtung nach einem der vorhergehenden Ansprüche, bei der das Gehäuse eine äußere Düse (7) an seinem geschlossenen Ende trägt, die nach außen den Konus begrenzt und dazu dient, beim Gebrauch eine getrennte Füll-, Misch- oder Ausgabeeinrichtung anzuschließen, um eine nach außen fluiddichte Strömungsverbindung zwischen der Ampulle und der getrennten Einrichtung über die Nadel und den Konus herzustellen.

11. Einrichtung nach Anspruch 10, bei der die Düse einen Luer-Verriegelungsanschluß (9) aufweist, der mit einem komplementären Verriegelungsanschluß an der getrennten Einrichtung zusammenpaßt, um eine lösbare Verbindung herzustellen.

12. Einrichtung nach einem der vorhergehenden Ansprüche, bei der der Sockel (34) Innengewindeeinrichtungen (55) enthält, die mit komplementären Außengewindemitteln (44) auf dem Stopfen der entsprechenden anzuschließenden Ampulle zusammenpassen.

13. Einrichtung nach einem der vorhergehenden Ansprüche, bei der das Gehäuse und die Hülse als Kunststoffspritzformteile zur einseitigen, frei auskragenden Rastverbindung hergestellt sind.

14. Zweiteilige Injektionseinrichtung (1) mit einem länglichen,hohlen äußeren Gehäuse (2), das ein vorderes geschlossenes Ende (7); ein hinteres offenes Ende (4); eine zylindrische Innenwand; die einen Innenraum bildet, der dazu dient, im Gebrauch eine zylindrische, mittels eines durchstoßbaren Stopfens (44) verschlossene Medikamentenampulle koaxial durch das offene Ende aufzunehmen und bei einer Einführbewegung relativ hierzu zu führen; eine zentrale Nabe (5), die von einem externen Nabenkonus (5a) durch das geschlossene Ende zu einem internen kreisförmigen Nabensitz (5b) in dem Inneren des Gehäuses führt und eine Bohrung (6) enthält, die von dem Konus bis zu dem Sitz hindurch führt, und das neben dem geschlossenen Ende des Gehäuseinneren eine kreisförmige Rastverbindungsanordnung (11, 12) zur frei auskragenden Verrastung aufweist, wobei die Innenwand,die Rastverbindungseinrichtung und der Sitz zueinander koaxial sind und
mit einer zylindrischen inneren Hülse (20), die zur einseitigen Befestigung ein vorderes Befestigungsende (21) mit einem Gegenstück zur kreisförmigen Rastverbindungseinrichtung (24); einen zentralen Nadelbefestigungsteil (28) und ein rückwärtiges freies Ende (22) aufweist, das einen zylindrischen Anschlußsockel (34) trägt, der dazu dient, den Stopfen daran stationär anzuschließen,sowie eine Nadel (32) umfaßt, die einen vorderen kreisförmigen Flanschkragen (33) sowie ein hinteres zugespitztes Ende aufweist, wobei die Hülse,das Gegenstück für die Rastverbindung, der Nadelhalterungsteil und der Sockel zueinander koaxial sind,
wobei die Hülse so bemessen ist, daß sie in dem Gehäuse mit ausreichendem radialen Abstand zu der Innenwand einzusetzen ist, damit ein Einführen der Ampulle zwischen diese möglich ist, die Rastanordnung und das Gegenstück für die Rastanordnung für eine Rastverbindung bemessen und angeordnet sind, um die Hülse koaxial in dem Gehäuse zu halten, indem das vordere Ende der Hülse mit dem geschlossenen Ende des Gehäuses stationär verbunden und die Hülse einenends eingespannt ist, und der Nadelbefestigungsteil dazu dient, die Nadel bezüglich der Bohrung und der Hülse koaxial und nach außen fluiddicht zu halten,
um den Flanschkragen mit der Bohrung strömungsmäßig zu verbinden und das zugespitzte Nadelende koaxial in dem Sockel zu halten, damit sie den Stopfen zur Herstellung einer geschützten, nach außen fluiddichten Strömungsverbindung zwischen der Ampulle und dem Konus über die Nadel und die Bohrung verbinden kann.

15. Zweiteilige Misch-, Spritzen-und Injektionseinrichtung mit einer zweiteiligen Injektionseinrichtung nach einem der vorhergehenden Ansprüche und einem Adapter (51), wobei jeder von ihnen ein vorderes geschlossenes und ein hinteres offenes Ende sowie einen geschützten Strömungsweg enthält, der sich in Längsrichtung von dem innen liegenden Ende zu dem äußeren Ende hindurch erstreckt;
wobei die zweiteilige Injektionseinrichtung eine externe Düse in ihrem vorderen geschlossenen Ende enthält;
wobei der Adapter in seinem vorderen geschlossenen Ende eine komplementäre Anschlußdüse (66) trägt, die ein innen liegendes Ende seines Strömungsweges bildet und die externe Düse und die komplementäre Anschlußdüse dazu dienen, lösbar die Injektionseinrichtung und den Adapter miteinander zu verbinden, um ihre Strömungswege geschützt und nach außen abgedichtet strömungsmäßig zu verbinden, wobei der Adapter eine sich von dem hinteren offenen Ende zu dem vorderen offenen Ende erstreckende Führungsbahn enthält, die dazu dient, beim Gebrauch eine zylindrische Medikamentenampulle, die ein oberes, von einem durchstoßbaren Stopfen verschlossenes Ende aufweist, aufzunehmen und bei einer Hindurchbewegung zu führen: und
mit einem Anschlußsockel (69) in dem hinteren Ende, der dazu dient, den durchstoßbaren Stopfen stationär anzuschließen sowie mit einem rohrförmigen Dorn (41), der in den Sockel hineinragt und an einer gegenüber dem offenen rückwärtigen Ende ausreichend zurückspringend endet, um den Dorn gegen eine unbeabsichtigte menschliche Berührung zu schützen, der dazu dient, bei der Verwendung durch den Stopfen hindurchzudringen, damit die Ampulle mit dem Strömungsweg nach außen abgedichtet verbunden ist.

16. Einrichtung nach Anspruch 15, bei der die Düse und das Düsengegenstück zusammenpassende Verriegelungseinrichtungen tragen, um die Injektionseinrichtung und den Adapter lösbar miteinander zu verbinden.

17. Einrichtung nach Anspruch 15 oder 16, bei der jeder Sockel eine axiale zylindrische Ausnehmung begrenzt und jeder Dorn einen axialen hohlen zylindrischen Rohrabschnitt begrenzt, der zentral angeordnet und gegenüber der zugehörigen Ausnehmung koaxial ist.

18. Einrichtung nach Anspruch 17, bei der jeder Dorn einenends gehaltert ist, ein zugespitztes freies Ende und ein Fußende aufweist, das mit dem benachbarten Teil des entsprechenden Endes der Injektionseinrichtung und des Adapters,durch den sich der entsprechende Strömungsweg erstreckt, integral ist.

19. Einrichtung nach Anspruch 18, bei der jeder Sockel mit einer Innengewindeanschlußeinrichtung versehen ist, die dazu dient, bei der Verwendung mit einer komplementären Außengewindeanschlußeinrichtung an dem Stopfen der betreffenden Ampulle, die anzuschließen ist, zusammenzupassen.

20. Einrichtung nach Anspruch 19, bei der das Gehäuse und die Hülse der zweiteiligen Injektionseinrichtung und des Adapters als Kunststoffspritzgußteile hergestellt sind.

## Revendications

1. Appareil injecteur (1) en deux parties, comprenant :
une enveloppe externe creuse et allongée (2) ayant une extrémité antérieure fermée (3), une extrémité arrière ouverte (4), une paroi interne ayant la forme d'un cylindre de section droite circulaire constituant une chambre à l'intérieur de l'enveloppe, un moyeu coaxial à la paroi interne et passant à travers l'extrémité fermée, depuis la chambre et jusqu'à une position avant externe à l'enve- loppe, un trou (6) traversant le moyeu, un siège coaxial (5b) entourant le trou à l'intérieur de la chambre, un bec (5a) formé par l'extrémité du moyeu à l'extérieur de l'enveloppe, et une première partie (11, 12) d'un ensemble de blocage par enclenchement élastique formé à l'extrémité de la paroi interne adjacente à l'extrémité fermée, et
un manchon interne (20) sous forme d'un cylindre de section droite circulaire, ayant une extrémité avant (21), une broche tubulaire montée coaxialement (55), et une extrémité arrière (22), une seconde partie (24) de l'ensemble de blocage par enclenchement élastique étant formée à l'extrémité avant du manchon interne pour que cette seconde partie coopère avec la première partie au montage du manchon interne en porte-à-faux à l'intérieur de la chambre, l'extrémité avant comportant un dispositif d'étanchéité destiné à coopérer de façon étanche avec le siège, lorsque le manchon est monté en position opérationnelle dans l'enveloppe, la broche ayant une extrémité pointue tournée vers l'arrière et se terminant vers l'intérieur par rapport à l'extrémité arrière ouverte de l'enveloppe externe, de manière suffisante pour que la broche soit protégée contre un contact humain intempestif, et un passage coaxial (27) de circulation formé entre la broche et le manchon interne, le diamètre externe dans la direction radiale du manchon étant inférieur au diamètre de la paroi interne si bien que, lorsque le manchon est monté en position opérationnelle dans l'enveloppe, un espace annulaire est formé entre la paroi interne et le manchon, le manchon ayant un logement de raccordement formé à son extrémité arrière, la broche dépassant dans ce logement,
l'appareil injecteur étant destiné à recevoir une ampoule cylindrique (40) de médicament à l'extrémité ouverte de l'enveloppe, l'ampoule ayant une paroi cylindrique creuse (41) dont l'épaisseur de paroi est inférieure à la distance radiale comprise entre la paroi interne et le manchon, l'ampoule pouvant coulisser par rapport à l'enve- loppe et étant guidée en déplacement par coopération de la paroi interne et du manchon, l'ampoule ayant une extrémité ouverte qui est fermée par un bouchon (42) qui peut être traversé, ce bouchon ayant une saillie dont la configuration et la forme permettent le raccordement avec le logement et la retenue par celui-ci, le bouchon pouvant coulisser axialement à l'intérieur de l'ampoule, la broche ayant une configuration telle que son extrémité pointue pénètre dans le bouchon lorsque l'ampoule est raccordée pendant le fonctionnement à l'appareil injecteur en permettant une circulation étanche entre l'ampoule et le bec par l'intermédiaire de la broche, du passage de circulation et du trou.

2. Appareil injecteur en deux parties selon la revendication 1, dans lequel la première partie de l'ensemble de blocage par enclenchement élastique est formée par une bague (11) à rétrécissement en saillie radiale vers l'intérieur formée près de l'extrémité avant de la paroi interne, et la seconde partie de l'ensemble de blocage à enclenchement élastique est formée par un flasque (23) de blocage en saillie radiale vers l'extérieur à l'extrémité avant du manchon, le flasque étant destiné à se bloquer par enclenchement sur la bague à rétrécissement afin que le manchon soit monté en porte-à-faux sur l'enveloppe lorsque le manchon est introduit dans l'enveloppe en cours, de fonctionnement.

3. Appareil injecteur en deux parties selon la revendication 1 ou 2, dans lequel la broche est sous forme d'une aiguille allongée (32) ayant un flasque (33) constituant un collier antérieur, et le manchon a une partie coaxiale de montage d'aiguille qui porte l'aiguille de manière étanche au fluide sur le manchon.

4. Appareil selon la revendication 2, dans lequel l'enveloppe a une paroi avant sensiblement radiale formant son extrémité antérieure fermée, et le flasque du manchon a une dimension et une disposition telles qu'il est en butée en position fixe contre la paroi avant lorsque le flasque est en coopération de blocage par enclenchement élastique avec la bague.

5. Appareil selon la revendication 3, dans lequel la partie de montage de l'aiguille comprend une cavité centrale circulaire tournée vers l'avant à l'extrémité avant du manchon, et un prolongement circulaire central tourné vers l'arrière, et le passage de circulation passe dans cette partie de la cavité vers le prolongement, le flasque formant un collier étant disposé pour être monté dans une direction coaxiale sur le prolongement, la cavité étant destinée à revevoir le siège dans une disposition coaxiale.

6. Appareil selon la revendication 5, dans lequel la partie de montage d'aiguille a un dispositif connecteur adjacent au prolongement, et le flasque formant un collier a un dispositif connecteur complémentaire destiné à raccorder l'aiguille au manchon au niveau du prolongement.

7. Appareil selon la revendication 3, dans lequel le manchon a une cavité annulaire (25) disposée radialement entre le flasque du manchon et la cavité et allant de l'extrémité avant du manchon vers le prolongement afin qu'un espace concentrique de compensation de compression soit délimité et facilite le déplacement local par compression du flasque dans la bague afin qu'elle se bloque par enclenchement élastique dans la gorge.

8. Appareil selon la revendication 3, dans lequel la partie de montage d'aiguille comporte un organe conformé central (26) sous forme d'une cavité circulaire, ayant un épaulement circulaire, l'organe conformé de la cavité, l'épaulement et le flasque étant coaxiaux, et l'organe conformé de la cavité et l'épaulement ayant une dimension permettant le logement et la retenue du flasque de collier alors que l'organe conformé de cavité entoure périphériquement le flasque de collier, et l'épaulement positionne axialement le flasque de collier afin que celui-ci soit monté directement sur le siège.

9. Appareil selon la revendication 8, dans lequel l'organe conformé de la cavité comporte une cavité circulaire centrale (260) de col de diamètre accru à l'extrémité avant du manchon, délimitée vers l'extérieur par une partie antérieure formant un col (250) à paroi mince dirigée vers l'arrière depuis le flasque du manchon, et une cavité circulaire centrale (280) de tige ayant un diamètre réduit et tournée vers l'arrière, délimitée vers l'extérieur par une partie constituant une tige (270) à paroi épaisse dirigée vers l'arrière de la partie formant col et séparée de celle-ci par l'épaulement, l'épaulement étant destiné à positionner axialement le flasque de collier en arrière de la partie la plus en avant de la partie de col, la partie de col ayant une dimension facilitant le déplacement du flasque par compression locale dans la bague afin qu'il assure le blocage dans la gorge par enclenchement élastique.

10. Appareil selon l'une quelconque des revendications précédentes, dans lequel l'enveloppe a une buse externe (7) à son extrémité fermée, délimitant à l'exté- rieur le bec et disposée, pendant l'utilisation, de manière qu'elle assure le raccordement d'un appareil séparé de chargement, de mélange ou de distribution et qu'un fluide puisse s'écouler de manière étanche entre une ampoule et l'appareil séparé par l'intermédiaire de l'aiguille et du bec.

11. Appareil selon la revendication 10, dans lequel la buse a un connecteur (9) de blocage "Luer" destiné à coopérer avec un raccord de blocage "Luer" complémentaire de cet appareil séparé afin qu'ils puissent être interconnectés de façon temporaire.

12. Appareil selon l'une quelconque des revendications précédentes, dans lequel le logement (34) a un taraudage (35) destiné à coopérer avec un filetage complémentaire (44) du bouchon de l'ampoule correspondante qui doit être raccordée.

13. Appareil selon l'une quelconque des revendications précédentes, dans lequel l'enveloppe et le manchon sont formés de pièces en matière plastique moulées par injection destinées à permettre un raccordement en porte-à- faux par enclenchement élastique.

14. Appareil injecteur (1) en deux parties, comprenant
une enveloppe longitudinale externe creuse (2) comportant une extrémité antérieure fermée (3), une extrémité arrière ouverte (4), une paroi cylindrique interne formant une partie interne qui, pendant l'utilisation, est destinée à recevoir dans une disposition coaxiale par son extrémité ouverte et à guider, afin qu'elle se déplace par rapport à elle, une ampoule cylindrique (40) de médicament fermée par un bouchon (42) qui peut être traversé, un moyeu central (5) passant par l'extrémité fermée depuis un bec externe (5a) du moyeu jusqu'à un siège circulaire interne (5b) du moyeu à l'intérieur de l'enveloppe et ayant un trou (6) qui le traverse du bec jusqu'au siège, et un organe conformé (11, 12) de connexion circulaire par enclenchement élastique et en porte-à-faux à l'intérieur de l'enveloppe près de l'extrémité fermée, la paroi interne, l'organe conformé de connexion et le siège étant coaxiaux, et
un manchon cylindrique interne (20) ayant une extrémité avant (21) de montage en porte-à-faux comportant un organe conformé circulaire complémentaire (24) de connexion monté en porte-à-faux et coopérant par enclenchement élastique, une partie centrale (28) de montage d'aiguille et une extrémité arrière libre (22) ayant un logement cylindrique (34) de connexion, destinée à assurer la connexion à demeure du bouchon, et une aiguille creuse (32) ayant un flasque circulaire avant (33) en forme de collier et une extrémité arrière pointue, le manchon, l'organe conformé complémentaire de connexion, la partie de montage d'aiguille et le logement étant coaxiaux,
le manchon ayant une dimension assurant le positionnement dans l'enveloppe en position suffisamment interne dans la direction radiale de la paroi interne pour que l'ampoule puisse être introduite, l'organe conformé de connecteur et l'organe conformé complémentaire de connecteur ayant une dimension et une disposition telles qu'ils peuvent coopérer par emboitement par enclenchement élastique en assurant le montage du manchon coaxialement dans l'enveloppe, l'extrémité avant du manchon étant raccordée à demeure en porte-à-faux à l'extrémité fermée de l'enveloppe, et la partie de montage d'aiguille étant destinée à supporter l'aiguille coaxialement au trou et en coopération étanche avec le trou et le manchon afin que le flasque en forme de collier permette la communication par circulation avec le trou, et que l'extrémité pointue de l'aiguille soit placée coaxialement dans le logement et puisse traverser le bouchon et permettre une circulation étanche protégée du fluide entre l'ampoule et le bec par l'intermédiaire de l'aiguille et du trou.

15. Appareil injecteur en deux parties et mélangeur à seringue, comprenant un injecteur en deux parties selon l'une quelconque des revendications précédentes et un adaptateur (51), ayant chacun une extrémité avant fermée et une extrémité arrière ouverte et un trajet protégé de fluide disposé longitudinalement entre l'extrémité interne et l'extrémité externe,
l'injecteur en deux parties comprenant une buse externe à son extrémité avant fermée,
l'adaptateur comprenant une buse complémentaire (66) de connexion formée à l'extrémité avant fermée, délimitant l'extrémité interne de son trajet, la buse externe et la buse complémentaire de connexion étant destinées à assurer l'interconnexion amovible de l'injecteur et de l'adaptateur afin que leurs trajets soient raccordés dans un état de circulation étanche et protégée, l'adaptateur ayant un guide allant de l'extrémité arrière ouverte à l'extrémité avant fermée et destiné, pendant l'utilisation, à recevoir et guider une ampoule cylindrique de médicament afin qu'elle puisse se déplacer par rapport à eux, l'ampoule ayant une extrémité supérieure fermée par un bouchon qui peut être traversé, et
un logement (69) de raccordement formé à l'extrémité arrière ouverte et destiné à raccorder à demeure le bouchon qui peut être traversé, et une broche tubulaire (71) dépassant dans le logement et aboutissant à l'intérieur de l'extrémité arrière ouverte de manière suffisante pour que la broche soit protégée contre un contact humain intempestif et, pendant l'utilisation, puisse pénétrer dans le bouchon afin qu'elle assure la connexion de l'ampoule au trajet en permettant la communication du fluide.

16. Appareil selon la revendication 15, dans lequel la buse et la buse complémentaire ont des organes conformés complémentaires de connexion à blocage "Luer" afin que l'injecteur et l'adaptateur soient interconnectés de manière amovible.

17. Appareil selon la revendication 15 ou 16, dans lequel chaque logement délimite une cavité cylindrique axiale et chaque broche délimite une partie tubulaire axiale cylindrique creuse disposée au centre coaxialement à sa cavité respective.

18. Appareil selon la revendication 17, dans lequel chaque broche est montée en porte-à-faux et a une extrémité libre pointue et une extrémité de base qui est solidaire de la partie adjacente de l'extrémité correspondante de l'injecteur et de l'adaptateur dans lequel se trouve le trajet respectif.

19. Appareil selon la revendication 18, dans lequel chaque logement a un organe conformé de raccordement taraudé destiné, pendant l'utilisation, à coopérer avec un organe conformé et complémentaire du bouchon d'une ampoule correspondante qui doit être raccordée.

20. Appareil selon la revendication 19, dans lequel l'enveloppe et le manchon de l'appareil injecteur en deux parties et de l'adaptateur sont formés de pièces de matière plastique moulées par injection.
